# EUROPEAN PATENT SPECIFICATION

(11) **EP 4 092 052 B1**
(45) Date of publication and mention of the grant of the patent: **12.03.2025**
(21) Application number: 21741624.7
(22) Date of filing: 05.01.2021
(51) Int. Cl.: C07K 17/10, C07K 17/08, G01N 33/68, G01N 33/569

(54) **LECTIN-MAGNETIC CARRIER CONJUGATE COMPLEX FOR ISOLATING GLYCOSYLATED EXOSOMES FROM CLINICAL SAMPLES**
LECTIN-MAGNETTRÄGERKONJUGATKOMPLEX ZUR ISOLIERUNG VON GLYKOSYLIERTEN EXOSOMEN AUS KLINISCHEN PROBEN
COMPLEXE CONJUGUÉ DE LECTINE-SUPPORT MAGNÉTIQUE DESTINÉ À ISOLER DES EXOSOMES GLYCOSYLÉS D'ÉCHANTILLONS CLINIQUES

(30) Priority: 19.01.2020 CN 202010060055
(43) Date of publication of application: 23.11.2022
(73) Proprietor: Beijing Glyexo Gene Technology Co., Ltd., Daxing District, Beijing 102600 (CN)
(72) Inventor: LIN, Changqing, Beijing 102600 (CN); CHEN, Tiansheng, Beijing 102600 (CN); HAO, Kun, Beijing 102600 (CN); HUANG, He, Beijing 102600 (CN); GAO, Qi, Beijing 102600 (CN); LI, Yanzhao, Beijing 102600 (CN); XU, Zhihui, Beijing 102600 (CN); QIE, Shuang, Beijing 102600 (CN); ZHAO, Guangzhen, Beijing 102600 (CN)
(74) Representative: LLR
(86) International application number: PCT/CN2021/070288
(87) International publication number: WO 2021/143576

(56) References cited:
- EP-A1- 3 242 133
- WO-A1-2011/066589
- WO-A1-2018/172384
- WO-A1-2018/207932
- CN-A- 109 266 599
- CN-A- 109 738 625
- CN-A- 111 253 491
- DOROTHY LOO ET AL: "Lectin Magnetic Bead Array for Biomarker Discovery", JOURNAL OF PROTEOME RESEARCH, vol. 9, no. 10, 1 October 2010 (2010-10-01), pages 5496 - 5500, XP055476508, ISSN: 1535-3893, DOI: 10.1021/pr100472z

## Description

### Cross-reference

The present invention claims the priority to Chinese Application No. 202010060055.7, entitled "Lectin-Magnetic Carrier Coupling Complex For Separating Glycosylated Exosomes From Clinical Sample", filed before the Patent Office of the People's Republic of China.

### Technical Field

The present invention belongs to the technical fields of biological medicines and bioseparation and relates to a lectin-magnetic carrier coupling complex for separating glycosylated exosomes from a clinical sample and a separation method.

### Background Art

Exosomes are small vesicles with biological activities, which are released when muhivesicular bodies are fused to plasma membranes, have diameters of about 30-150 nm and are one of important media for intercellular communication. Under a normal physiological condition of human body, exosomes may deliver various bioactive substances such as DNA, proteins, mRNA, and miRNA among cells, and participate in various processes such as cell communication, cell migration, and tumor cells growth so as to complete intercellular delivery of substances and information. Literature researches have reported that nearly all types of cells can secrete the exosomes, which can be extracted from body fluids such as blood, saliva, urine, cerebrospinal fluids, hydrops, and breast milk.

Exosomes are related to many diseases including tumors, chronic infectious diseases, and autoimmune diseases. When a body is diseased, the composition, content, and properties and the like of exosomes secreted by the cells may change. Glycosylated exosomes play an important role in the occurrence and development of various diseases including tumors; and detection of exosomes can effectively monitor the occurrence and development of diseases. Due to a relatively low content of exosomes in a clinical sample, separation and enrichment of the exosomes are crucial to researches on the exosomes.

At present, there are many separation methods for the exosomes, mainly including ultracentrifugation or density-gradient centrifugation and an immunomagnetic separation method and the like. The ultracentrifugation or the density-gradient centrifugation method conducts the separation based on small differences in density between the exosomes and other biological components, so that these methods require special equipment(s) and large amounts of samples and are time-consuming; and as different clinical samples have different characteristics, the method has a poor stability in the separation effect, and further, the ultracentrifugation method is also easy to damage vesicles, so as to affect subsequent experiment(s). The principle of the immunomagnetic separation method is that specific proteins on the surfaces of the exosomes, such as CD9, CD63, and CD81, bind to immunomagnetic beads coated with the corresponding antibodies for magnetic separation; however, the immunomagnetic beads are relatively small in size and have nano-scale particle sizes, which are smaller than or equal to the diameters of the exosomes, so that steric hindrance of the immunomagnetic beads binding to the exosomes is relatively large, causing that the binding to the exosomes is insufficient, and the separation efficiency thereof is low. In addition, since the elution buffer applied in the immunomagnetic separation method is acid and is easy to make vesicles of the exosomes broken and incomplete in morphology, glycosylated exosomes cannot be accurately separated and thereby cannot be applied for detection, monitoring, or diagnosis on occurrence, development, and other processes of diseases.

The document Leo et al, "Lectin magnetic bead array for biomarker discovery", Journal of proteome research, vol. 9, no. 10, 1 October 2010, pages 5496-5500, teaches the use of lectin-couples agarose magnetic beads and washing/binding buffer containing TRIS for the isolation of glycosylated proteins.

Patent document WO 2018/172384 to Pfaffl, M. et al (27-09-2018) discloses methods for isolating and quantifying exosomes in a sample using magnetic latex beads, washing buffers containing metal salt ions, and elution buffers comprising a saccharide solution.

Therefore, it is necessary to develop and establish a composition and a method which can be used for rapidly, accurately, automatically, and efficiently separating and enriching glycosylated exosomes in a clinical sample.

### Summary of the Invention

The invention is defined in the appended claims.

To address the deficiencies in the prior art, the present invention provides a lectin-magnetic carrier coupling complex for separating glycosylated exosomes from a clinical sample, and a composition for separating glycosylated exosomes containing the lectin-magnetic carrier coupling complex, and a method of separating glycosylated exosomes by using the lectin-magnetic carrier coupling complex and use thereof. The lectin-magnetic carrier coupling complex provided herein may accurately separate glycosylated exosomes from a clinical sample and achieve a high separation efficiency; and the separated exosomes are intact in morphology without rupturing or cracking, may be directly used for liquid detection of glycosylated exosomes, or directly used for immunology-related detection, or directly used for gene detection or analysis after extracting related nucleic acids from the exosomes. The method of separating glycosylated exosomes provided by the present invention takes the principle that sugar chains rich on the surfaces of the exosomes may bind to lectins, and uses effective washing and effective elution method to separate glycosylated exosomes, which is very simple, convenient, rapid, and easy to achieve automation, and has a good repeatability; and the separated exosomes are intact in morphology without rupturing or cracking, and can be applied to detection, monitoring, and diagnosis on occurrence, development, and other processes of diseases.

Provided herein is a lectin-magnetic carrier coupling complex for separating glycosylated exosomes from a clinical sample, comprising a magnetic carrier and lectins coupled to the outer side of the magnetic carrier, wherein
the lectins are any one type of Artocarpus integrifolia lectin, peanut lectin, Pisum sativum lectin (VVA and/or VVL), Concanavalin lectin, Lens culinaris lectin, wheat germ lectin, soybean lectin, kidney bean lectin, and snail lectin (HAA and/or HPA), or a combination of two or more of the above;
the magnetic carrier is any one type of a dextran magnetic bead, an agarose magnetic bead, a resin or epoxy resin magnetic bead, and a polystyrene magnetic bead, or a combination of two or more of the above.

In some embodiments, for the lectin-magnetic carrier coupling complex, a particle size distribution range of the magnetic carrier is from 1 µm to 200 µm, preferably, from 10 µm to 200 µm, and more preferably, from 30 µm to 150 µm.

In some embodiments, in the lectin-magnetic carrier coupling complex, 1-20 mg, preferably, 5-15 mg, and more preferably, 10-15 mg of lectins are coupled to each l mL of the magnetic carrier. After coupling of the lectins to the magnetic carrier, the resultant is treated with a bovine serum albumin buffer without exosomes to obtain a final lectin-magnetic carrier coupling complex.

In some embodiments, for the lectin-magnetic carrier coupling complex, the clinical sample is any one of serum, plasma, saliva, a tissue or cell culture supernatant, urine, a cerebrospinal fluid, and a lymph fluid.

In some embodiments, for the lectin-magnetic carrier coupling complex, the lectins are any one type of Artocarpus integrifolia lectin, peanut lectin, Pisum sativum lectin (VVA and/or VVL), Concanavalin lectin, Lens culinaris lectin, wheat germ lectin, soybean lectin, and kidney bean lectin, or a combination of two or more of the above.

In some embodiments, for the lectin-magnetic carrier coupling complex, the glycosylated exosomes are any one type of N-glycosylated exosomes, O-glycosylated exosomes, and fucosylated exosomes, or a combination of two or more of the above.

In some embodiments, the lectin-magnetic carrier coupling complex is preserved in a preservation solution, and the preservation concentration (volume ratio) of the lectin-magnetic carrier coupling complex in the obtained lectin-magnetic carrier coupling complex preservation solution is 10%-60%, preferably, 30%-50%, and more preferably, 40%-50%; and the volume of the lectin-magnetic carrier coupling complex preservation solution refers to a total volume of the lectin-magnetic carrier coupling complex, after being dispersed in the preservation solution, and the preservation solution.

In another aspect, the present invention further provides a composition for separating glycosylated exosomes, comprising the lectin-magnetic carrier coupling complex, a washing buffer for washing and for removing the exosomes nonspecifically bound to the coupling complex or not glycosylated and other impurities in the separation process, and an elution buffer for eluting the glycosylated exosomes specifically bound to the lectin-magnetic carrier coupling complex. Here, the composition does not refer to mixing the above components together, but refers to separately packaging of the above components and co-existence of the above components in one suite at the same time.

For the composition for separating glycosylated exosomes, the washing buffer is a metal salt ion-free washing buffer or purified water, and optionally, is a metal salt ion-free washing buffer, and further optionally, is a metal salt ion-free washing buffer with a pH value of 7.6±0.2, which is used for washing and for removing the exosomes nonspecifically bound to the coupling complex or not glycosylated and other impurities in the separation process.

For the composition for separating glycosylated exosomes, the elution buffer is a borate buffer with saccharides dissolved therein, optionally, is a borate buffer with mannose dissolved therein (i.e. borate-mannose buffer), and further optionally, is a borate-mannose buffer with a pH value of 6.5±0.2, which is very close to a physiological pH value; the elution buffer is used for eluting the glycosylated exosomes bound to the lectin-magnetic carrier coupling complex; and the eluted exosomes are intact in morphology without rupturing or cracking.

In another aspect, the present invention further provides a method of separating glycosylated exosomes, comprising a separation step of using the above lectin-magnetic carrier coupling complex to separate glycosylated exosomes. The separation step comprises:
pretreatment of a clinical sample:
fully and uniformly mixing the pretreated sample with the lectin-magnetic carrier coupling complex for incubation;
conducting separation on the lectin-magnetic carrier coupling complex bound to the glycosylated exosomes by means of magnetic separation, and washing the lectin-magnetic carrier coupling complex bound to the glycosylated exosomes with a washing buffer; and
eluting the washed lectin-magnetic carrier coupling complex with an elution buffer. The elution supernatant is a solution of the separated glycosylated exosomes; and the eluted exosomes are intact in appearance and morphology without rupturing or cracking.

In some embodiments, for the method of separating glycosylated exosomes, a volume ratio of the pretreated sample to the lectin-magnetic carrier coupling complex is (0.5-5): 1, preferably, is (0.5-3): 1, and more preferably, is (1-2): 1.

In some embodiments, for the method of separating glycosylated exosomes, the washing step is performed with the washing buffer 1-3 times the volume of the lectin-magnetic carrier coupling complex for 1-3 times.

In some embodiments, for the method of separating glycosylated exosomes, a volume ratio of the elution buffer to the lectin-magnetic carrier coupling complex is (0.5-2): 1, and preferably, is (0.5-1): 1.

In some embodiments, for the method of separating glycosylated exosomes, the pretreatment of the sample comprises the following steps:
for a serum sample, a plasma sample, a saliva sample, a cerebrospinal fluid sample, or a lymph fluid sample, subjecting the sample to centrifugation at a speed of 3000 g or below for 10-15 min, to remove cell debris, precipitates, and other impurities in the sample, and taking the supernatant after centrifugation for later use; and
for a tissue or cell culture supernatant sample or a urine sample, subjecting the sample to centrifugation at a speed of 3000 g or below for 10-15 min, to remove cell debris, precipitates, and other impurities in the sample, and then concentrating the supernatant by 10-1000 times through an ultrafiltration tube for later use.

For the method of separating glycosylated exosomes, the washing buffer is a metal salt ion-free washing buffer or purified water, optionally, is a metal salt ion-free washing buffer, and further optionally, is a metal salt ion-free washing buffer with a pH value of 7.6±0.2.

For the method of separating glycosylated exosomes, the elution buffer is a borate buffer with saccharides dissolved therein, optionally, is a borate buffer with mannose dissolved therein (i.e. borate-mannose buffer), and further optionally, is a borate-mannose buffer with a pH value of 6.5±0.2.

In some embodiments, the incubation is conducted under the conditions of: at the room temperature for 1-30 min, preferably, for 5-20 min, and more preferably, for 10-15 min.

Also described herein is the use of exosomes separated by using the above method, comprising uses for liquid detection of glycosylated exosomes, for immunological detection of the exosomes, or for detection or analysis of nucleotide fragments of nucleic acids extracted from the exosomes.

### Beneficial effect:

1. For the lectin-magnetic carrier coupling complex described herein, the exosomes obtained by separation with the coupling complex are all glycosylated exosomes with intact morphology and without rupturing, may be directly used for liquid detection of glycosylated exosomes, immunological detection of the exosomes, or detection or analysis of nucleotide fragments of nucleic acids extracted from the exosomes, and the like, and may further achieve rapid, accurate, and automatic separation of glycosylated exosomes in cooperation with corresponding equipments.
2. In the lectin-magnetic carrier coupling complex described herein, the magnetic carrier has a particle size of 10-1000 times that of a common immunomagnetic bead; and a single magnetic bead has a larger surface area, so that steric hindrance of the lectins being coupled to the magnetic bead is decreased, facilitating to couple more lectins. In contrast, although the same volume of a common immunomagnetic bead has larger specific surface area, less lectins will be coupled to the common immunomagnetic bead due to larger steric hindrance. In the lectin-magnetic carrier coupling complex of the present invention, 1-20 mg of lectins may be coupled to each 1 mL of immunomagnetic bead; whereas researchers have discovered that when the immunomagnetic bead is used in a smaller volume, the amount of the lectins coupled to each 1 mL of immunomagnetic bead is in an ng scale, indicating low coupling efficiency. Meanwhile, the particle size of the magnetic carrier is also far larger than the diameters of the exosomes, so that steric hindrance of the lectins binding to the glycosylated exosomes may be decreased, thereby facilitating the binding of the lectins to the glycosylated exosomes, and greatly improving the separation efficiency of the exosomes.
3. The lectin-magnetic carrier coupling complex described herein has good effect in separating exosomes from a clinical sample containing less exosomes, such as urine or a tissue or cell culture supernatant; and when used for separating the exosomes in a clinical sample containing more exosomes, such as plasma, the lectin-magnetic carrier coupling complex can separate a concentration of up to 10¹¹⁻¹⁴/mL of the glycosylated exosomes.
4. In the lectin-magnetic carrier coupling complex described herein, the used lectins are preferably phytolectins, including Artocarpus integrifolia lectin, peanut lectin, Pisum sativum lectin (VVA and/or VVL), Concanavalin lectin, Lens culinaris lectin, wheat germ lectin, soybean lectin, and kidney bean lectin; and the phytolectins are more easily available in a great amount and raw materials are more abundant.
5. For the composition for separating glycosylated exosomes according to the present invention, the metal salt ion-free washing buffer or purified water is used; metal salt ions have a dissociation effect on affinity adsorption between the lectins and the glycosylated exosomes, which affects the affinity adsorption between the lectins and the glycosylated exosomes; and in addition, the higher the concentration of the metal salt ions is, the stronger the dissociation effect is, so that the separation effect of the exosomes is impaired, and even that the exosomes cannot be separated. By using the metal salt ion-free washing buffer or the purified water, it can be avoided that the exosomes obtained by the separation are reduced or even cannot be separated due to the impairment of the metal salt ions on the binding of the exosomes to lectins on the surface of the magnetic beads when the metal salt ion-free washing buffer or the purified water is used for washing.
6. For the composition for separating glycosylated exosomes according to the present invention, the used elution buffer is the borate buffer; compared with other buffers, the borate buffer has better elution effect on the exosomes, exhibiting high elution efficiency, and elution may be accomplished with a small amount of the elution buffer; the eluted exosomes are intact in appearance and morphology without rupturing or cracking; and meanwhile, due to less usage amount, concentration of exosomes may be accomplished at the same time.
7. For the method of separating glycosylated exosomes according to the present invention, the pretreatment of the sample is simple and feasible; and the sample suitable for the present invention may be effectively obtained through simple centrifugation and/or concentration without centrifuging the sample at an ultra high speed, and rapid, accurate, and automatic separation of glycosylated exosomes may be achieved by matching with corresponding equipments, and the glycosylated exosomes in the sample may be protected to the maximum extent.

### Brief Description of the Drawings

FIG. 1 is a schematic diagram of separation of glycosylated exosomes by using a lectin-magnetic carrier coupling complex.
FIG. 2 is a set of electron microscopic images showing the separation of glycosylated exosomes from 4 different types of samples (cell culture supernatant, plasma, urine, and cerebrospinal fluid).
FIG. 3 is a NTA analysis diagram of glycosylated exosomes separated from a plasma sample.
FIG. 4 is a diagram showing results of Western Blot analysis of exosomes separated from 4 different types of samples (cell culture supernatant, plasma, urine, and cerebrospinal fluid) with antibodies against exosome-specific membrane proteins CD9, CD63, and CD81.
FIG. 5 is a diagram showing results of Western Blot analysis of exosomes separated from 4 different types of samples (cell culture supernatant, plasma, urine, and cerebrospinal fluid) with antibodies against exosome-specific non-membrane proteins ALIX and TSG101.
FIG. 6 is a diagram showing results of Western Blot analysis of exosomes separated from 4 different types of samples (cell culture supernatant, plasma, urine, and cerebrospinal fluid) with an anti-Calnexin antibody.
FIG. 7 is a diagram showing results of Western Blot analysis of glycosylated exosome solutions with antibodies against exosome-specific membrane proteins CD9, CD63, and CD81, wherein the glycosylated exosome solutions were obtained by washing the lectin-magnetic carrier coupling complexes bound to glycosylated exosomes with 5 different washing buffers, respectively, and then eluting the same.
FIG. 8 is a set of electron microscopic images of solutions of glycosylated exosomes separated by 3 different elution buffers, respectively.

### Detailed Description of the Invention

In order to better describe the present invention, various illustrative embodiments of the present invention are described in detail now. The details description should not be considered as limiting to the present invention and should be understood as more detailed description of certain aspects, features, and embodiments of the present invention.

Terms "comprising", "including", "having", "containing" and the like used herein are open terms, that is, the terms mean including, but not limited to. Unless expressly specified otherwise, various reagents used below are all commercial reagents, wherein used chemical reagents are analytically pure.

### Example 1. Preparation of Lectin-Magnetic Carrier Coupling Complex

A lectin-magnetic carrier coupling complex comprises a magnetic carrier, and lectins coupled to the outer side of the magnetic carrier. The lectin-magnetic carrier coupling complex is prepared by coupling the lectins to the magnetic carrier under specific conditions and is mainly used for separating glycosylated exosomes in a clinical sample. The glycosylated exosomes obtained by the separation are intact in morphology, wherein:
the lectins are mainly selected from any one of Artocarpus integrifolia lectin (Jacalin), peanut lectin (PNA), Pisum sativum lectin (VVA and/or VVL), Concanavalin lectin (ConA), Lens culinaris lectin (LCA), wheat germ lectin (WGA), soybean lectin (SBA), kidney bean lectin (PVL), and snail lectin (HAA and/or HPA), or two or more of the above lectins used in combination; the main reason for selecting two or more lectins used in combination is: this combination of different lectins may achieve separation of various types of glycosylated exosomes, such as separation of fucosylated exosomes with LCA and AAL, separation of N-glycosylated exosomes with lectins such as ConA, PVL, SBA, WGA, or AAL, and separation of O-glycosylated exosomes with lectins such as HAA, HPA, VVA, PNA, or Jacalin; and a single type of lectins are mainly used for separating a specific type of glycosylated exosomes corresponding to the lectins, whereas two or more of the lectins used in combination may be used for separating glycosylated exosomes in various forms, or two or more of the lectins used in combination may be used for synergistically separating the specific glycosylated exosomes.

For further illustration of the present invention, the following examples mainly use the Lens culinaris lectin (LCA) (purchased from Sigma Company, America) for further description of the present invention.

The magnetic carrier mainly refers to a composite magnetic bead with magnetic separation effect and the surface of which is coated with polymer materials. The magnetic carrier is any one of a dextran magnetic bead, an agarose magnetic bead, a resin or epoxy resin magnetic bead, and a polystyrene magnetic bead, or a mixture of two or more of the above magnetic carriers. A particle size distribution range of the magnetic carriers is from 1 µm to 200 µm, preferably, from 10 µm to 200 µm, and more preferably, from 30 µm to 150 µm. In the production of the magnetic carriers, the particle sizes of the magnetic carriers produced in the same batch are usually not uniform. Therefore, the particle sizes of the magnetic carriers may be usually described either in the average particle size, or in a particle size distribution range as adopted in the present invention. For the description manner in the particle size distribution range, it may be considered that the specific particle size distribution conforms to or substantially conforms to the normal distribution within this distribution range. In the lectin-magnetic carrier coupling complex of the present invention, the particle size of the used magnetic carrier is 10-1000 times that of the common immunomagnetic beads; and a single magnetic carrier has a larger surface area, so that steric hindrance of the lectins being coupled to the magnetic carrier is decreased, facilitating to couple more lectins. In contrast, although the same total volume of common immunomagnetic carriers has larger specific surface area, less lectins are coupled to the magnetic carrier due to larger steric hindrance. In the lectin-magnetic carrier coupling complex of the present invention, 1-20 mg of lectins may be coupled to each 1 mL of magnetic carrier; whereas researchers have discovered that when the magnetic carrier is used in a smaller volume, the amount of the lectins coupled to each 1 mL of immunomagnetic carrier is in an ng scale, indicating low coupling efficiency. Meanwhile, the particle size of the magnetic carrier is also far larger than the diameters of the exosomes, so that steric hindrance of the lectins binding to the glycosylated exosomes may be decreased, thereby facilitating the binding of the lectins to the glycosylated exosomes, and greatly improving the separation efficiency of the exosomes.

The above magnetic carrier can couple to lectins through polymer coating materials on its surface to form the lectin-magnetic carrier coupling complex. Different types of magnetic carriers can all achieve the effect of coupling to the lectins by adjusting steps of soaking, coupling, washing, preservation, and the like. Certainly, for different types of lectins, the most suitable polymer materials may be different. In the preparation process of the lectin-magnetic carrier coupling complex, a ratio of the magnetic carrier (mL) to the lectins (mg) is (1:1)-(1:20), preferably, is (1:5)-(1:15), and more preferably, is (1:10)-(1:15).

The prepared lectin-magnetic carrier coupling complex is preserved in a preservation solution; and the preservation concentration (volume ratio) of the lectin-magnetic carrier coupling complex in the lectin-magnetic carrier coupling complex preservation solution is 10%-60%, preferably, is 30%-50%, and more preferably, is 40%-50%.

For further description of the present invention, the following examples all employ agarose magnetic beads with a particle size distribution range from 30 µm to 150 µm. The agarose magnetic beads are commercial NHS-activated agarose magnetic beads (purchased from Enriching biotechnology) with a volume concentration of 10%.

**The preparation process of the lectin-magnetic carrier coupling complex, i.e. LCA-agarose magnetic bead coupling complex was as follows in detail:**
(1) the commercial agarose magnetic beads were uniformly mixed, 50 mL of the uniformly mixed agarose magnetic beads were added to a 200 mL vessel, and magnetic separation was conducted to remove the supernatant and to obtain 5 mL of agarose magnetic beads.
(2) 100 mL of anhydrous ethanol was added, followed by uniformly mixing, and magnetic separation was conducted to remove the supernatant, which steps were repeated for 1-3 times to achieve the purpose of washing the agarose magnetic beads.
(3) Lens culinaris lectin and the agarose magnetic beads obtained in Step (2) were added to 50 mL of 0.1-1 M MES buffer (containing 0.1-0.5 M NaCl) with a pH value of 6-7, followed by uniformly mixing for later use, wherein the ratio of the volume (mL) of the agarose magnetic beads to the weight of the Lens culinaris lectin (LCA, mg) is (1:1)-(1:20) (that is, each 1 mL of agarose magnetic beads being mixed with 1-20 mg of Lens culinaris lectin). For better description of the present invention, in this example, the MES buffer has a concentration of 0.1 M and a pH value of 6.5 and contains 0.1 M NaCl; and the ratio of the volume (mL) of the agarose magnetic beads to the weight of the Lens culinaris lectin (LCA, mg) is 1: 10, that is, 5 mL of the agarose magnetic beads and 50 mg of LCA were added to 50 mL of 0.1 M MES buffer.
(4) The solution obtained in Step (3) was allowed to react under shaking at room temperature for 0.5-5 h, and magnetic separation was conducted to remove the supernatant, in which lectins could not be detected substantially. For better description of the present invention, in this example, shaking was conducted at the room temperature for 3 h.
(5) A bovine serum albumin buffer without the exosomes were added for blocking, that is, 200 mL of 0.1-1 M MES-B buffer with the pH value of 6-7 without the exosomes (containing 0.1-0.5 M NaCl and 0.1-1% BSA solution) was added, followed by shaking at the room temperature for 0.5-5 h, and then magnetic separation was conducted to remove the supernatant. For better description of the present invention, in this example, 200 mL of 0.5 M MES-B buffer with the pH value of 6.5 (containing 0.3 M NaCl and 0.5% BSA solution) was added, and shaking was conducted at the room temperature for 3 h.
(6) 10-200 mM TRIS buffer was added, followed by shaking at the room temperature for uniform mixing, and magnetic separation was conducted to remove the supernatant, which steps were repeated for 1-5 times. For better description of the present invention, in this example, 100 mM TRIS buffer was added.
(7) the LCA-agarose magnetic beads collected in Step (6) were resuspended in the TRIS buffer to formulate 10%-60% LCA-agarose magnetic bead solution for later use, and preferably, to formulate 30%-50% LCA-agarose magnetic bead solution. In this example, 5 mL of TRIS buffer was added to prepare the LCA-agarose magnetic bead solution with a volume ratio of 50%.

### Example 2. Preparation of Composition for Separating Glycosylated Exosomes

The present invention further provides a composition for separating glycosylated exosomes, comprising the lectin-magnetic carrier coupling complex, a washing buffer for washing and for removing the exosomes nonspecifically bound to the complex or not glycosylated and other impurities in the separation process, and/or an elution buffer for eluting the glycosylated exosomes specifically bound to the lectin-magnetic carrier coupling complex. The above components are separately packaged and existed in one suite or kit at the same time.

In this example, the lectin-magnetic carrier coupling complex was the LCA-agarose magnetic bead solution with the volume ratio of 50%, obtained in Example 1.

The washing buffer was a metal salt ion-free washing buffer or purified water, and optionally, was a metal salt ion-free washing buffer, for example, 10-200 mM metal salt ion-free TRIS-HCl buffer with a pH value of 7.6±0.2; and the washing buffer was used for washing and for removing the exosomes nonspecifically bound to the complex or not glycosylated and other impurities in the separation process. In this example, the washing buffer was 100 mM metal salt ion-free TRIS-HCl buffer with the pH value of 7.6±0.2.

The elution buffer was a borate buffer with saccharides dissolved therein and optionally was a borate buffer with mannose dissolved therein, for example, 10-20 mM borate buffer with 100-500 mM mannose dissolved therein and with a pH value of 6.5±0.2; the borate buffer was used for eluting the glycosylated exosomes bound to the lectin-magnetic carrier coupling complex; and the eluted exosomes were intact in morphology without rupturing or cracking. In this example, the elution buffer was 15 mM borate buffer with 300 mM mannose dissolved therein and with a pH value of 6.5±0.2.

### Example 3. Use Method of the Composition for Separating Glycosylated Exosomes

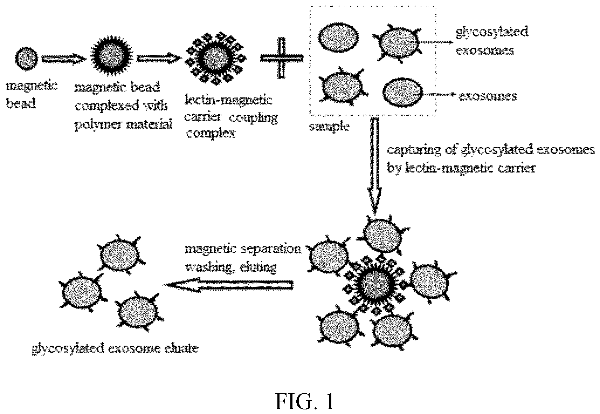

The present invention further provides a method for separating glycosylated exosomes, which mainly comprises the main experimental step of using the composition for separating glycosylated exosomes recited in Example 2. The separation principle may refer to FIG. 1 "a schematic diagram of separation of glycosylated exosomes by using a lectin-magnetic carrier coupling complex" in detail.

The detailed experimental procedure of the present invention comprises:

### 1. Preparation before experiment

Autonomous preparation of apparatuses or equipment: a magnetic frame, used for manually separating glycosylated exosomes; or a full-automatic agarose magnetic separating instrument from the Group Company and subsidiaries, mainly used for fully automatically separating the glycosylated exosomes to achieve the purpose of saving manpower. Full-automatic separation of the glycosylated exosomes with the agarose magnetic beads is just an automatic implementation for a manual mode and can achieve an effect equivalent to or superior to manual separation. This example employs the magnetic frame with manual separation for further illustration.

### 2. Experimental procedure

(1) pretreatment of a clinical sample: a method of pretreatment of the sample can be adjusted according to the characteristics of different clinical samples.
   For a serum sample, a plasma sample, a saliva sample, a cerebrospinal fluid sample, or a lymph fluid sample, the sample was subjected to centrifugation at 3000 g for 10-15 min to remove cell debris, precipitates, and other impurities in the sample, and the supernatant after centrifugation was transferred into a vessel for magnetic separation, such as a centrifuge tube, for later use.
   For a tissue or cell culture supernatant sample or a urine sample, the sample was subjected to centrifugation at 3000 g for 10-15 min to remove cell debris, precipitates, and other impurities in the sample, and then the supernatant was concentrated by 10-1000 times through an ultrafiltration tube, and then the concentrated supernatant was transferred into a vessel for magnetic separation, such as a centrifuge tube, for later use;
(2) 50-500 uL, optionally, 50-300 uL, further optionally, 100-200 uL, more optionally, 100 uL of the pretreated sample obtained in Step (1) was fully and uniformly mixed with 100 uL of lectin-magnetic carrier coupling complex for incubation, wherein the above volumes of the pretreated sample and of the lectin-magnetic carrier coupling complex preservation solution just embody their mixing ratio, but not represent their fixed volumes; and those skilled in the art may increase and decrease the volumes in a specific uniform mixing according to the ratio certainly. Certainly, the specific volumes of the pretreated sample and of the lectin-magnetic carrier coupling complex to be added to the vessel for magnetic separation are required to adapt to the volume of the vessel for magnetic separation. The preservation concentration (volume ratio) of the lectin-magnetic carrier coupling complex in the lectin-magnetic carrier coupling complex preservation solution was 10-60%, preferably, 30-50%, more preferably, 40-50%. The incubation was performed under conditions of: at the room temperature for 1-30 min, preferably, 5-20 min, more preferably, 10-15 min. When selecting different ratios and different incubation periods at the room temperature, the separation effects might be slightly different, but the same separation effect can be achieved substantially. For further description of the present invention, this example mainly employed the following steps: 200 uL of LCA-agarose magnetic bead preservation solution (comprising 100 uL of LCA-agarose magnetic beads) was placed in a 1.5 mL centrifuge tube, to which 100 uL of the pretreated sample was added for fully and uniformly mixing, and then the centrifuge tube was uniformly shaked at the room temperature for incubation for 10-15 min;
(3) the lectin-magnetic carrier coupling complex bound to the exosomes was separated by using the magnetic frame, and the resultant was washed for 1-3 times with a metal salt ion-free washing buffer with a pH value of 7.6±0.2, in a volume 1-3 times that of the lectin-magnetic carrier coupling complex, for washing and for removing the exosomes nonspecifically bound to the complex or not glycosylated and other impurities in the separation process. In this example, the detailed steps comprised: the centrifuge tube in Step (2) was placed on the magnetic frame for still standing for 1 min, and then the supernatant was discarded; at this time, the glycosylated exosomes were adsorbed onto the LCA-agarose magnetic bead complex; for 100 uL of LCA-agarose magnetic beads, 200 uL of metal salt ion-free washing buffer, which was 100 mM metal salt ion-free TRIS-HCl buffer, with a pH value of 7.6±0.2 was added; after fullly and uniformly mixing by suction, the mixture was placed on the magnetic frame for still standing for 1 min, and then the supernatant was discarded; and the washing procedure was repeated for 3 times;
(4) a borate-mannose buffer with a pH value of 6.5±0.2, serving as an elution buffer, was used to elute the washed lectin-magnetic carrier coupling complex, wherein the borate-mannose buffer mainly comprised 10-20 mM borate buffer and 100-500 mM mannose dissolved therein, and for 100 uL of LCA-agarose magnetic beads, the usage volume of the elution buffer was 50-200 uL, preferably, 50-100 uL; and the elution supernatant was namely a solution of the separated glycosylated exosomes, wherein the eluted exosomes were intact in appearance and morphology without rupturing or cracking. In this example, the detail steps comprised: 100uL of an elution buffer for glycosylated exosomes, which comprised 15 mM borate buffer and 300 mM mannose dissolved therein, was added to the product obtained in Step (3); after uniformly mixing by suction, the mixture was placed on the magnetic frame for still standing for 1 min or above; and the supernatant was taken and placed in a new centrifuge tube to be directly used for detection or to be preserved at -80±5 °C for later use; it should be noted that magnetic beads and impurities were not sucked when taking the supernatant; the eluted and separated glycosylated exosomes were intact in morphology and could be directly used for liquid detection of glycosylated exosomes, immunological detection of exosomes, gene detection and/or analysis after nucleic acid extraction from exosomes, and the like.

### Example 4

This example mainly makes further illustration for samples from which glycosylated exosomes are to be separated. Samples which can be used for the present invention may be selected from any one of serum, plasma, saliva, a tissue or cell culture supernatant, urine, a cerebrospinal fluid, and a lymph fluid. For a conventional 1.5 mL centrifuge tube, 50-500 uL, preferably, 50-300 uL, more preferably, 100-200 uL of the pretreated serum, plasma, saliva, cerebrospinal fluid, lymph fluid, tissue or cell culture supernatant, or urine sample could be added thereto. In the pretreatment process of the sample, the serum sample, the plasma sample, the saliva sample, the cerebrospinal fluid sample, or the lymph fluid sample was subjected to centrifugation only, and therefore, a desired volume of the pretreated sample might be obtained by pretreating 50-500 uL, preferably, 50-300 uL, more preferably, 100-200 uL of the sample; whereas in the pretreatment process of the tissue or cell culture supernatant sample or the urine sample, in addition to the centrifugation step, it was further required to concentrate the sample by 10-1000 times, and therefore, in order to obtain sufficient amount and optionally a desired volume of the pretreated sample, it was required to subject 1-50 mL, optionally, 10-50 mL, further optionally, 30-50 mL of the tissue or cell culture supernatant sample or the urine sample to the pretreatment.

Different volumes of the sample to be separated have little effect on the separation effect, but can all achieve the effect of separating glycosylated exosomes substantially. For further illustration of the present invention, in this example, according to the separation step in Example 3, 100 uL of the pretreated serum sample, the pretreated plasma sample, the pretreated saliva sample, the pretreated cerebrospinal fluid sample, or the pretreated lymph fluid sample was selected for illustration; and 50 mL of the sample to be separated and 100 uL of the pretreated sample upon concentration were selected for illustration of the tissue or cell culture supernatant sample or the urine sample.

According to the specificities of the to-be-separated samples of cell culture supernatant, serum, plasma, cerebrospinal fluid, urine, hydrops, breast milk, saliva, lymph fluid or the like, 4 types of samples, including the cell culture supernatant sample, the plasma sample, the urine sample, and the cerebrospinal fluid sample, were selected for further illustration; and for other types of samples than the above ones, the same separation effect can also be achieved. According to the procedure in Example 3, glycosylated exosomes were separated from 100 uL of the pretreated cell culture supernatant sample, plasma sample, urine sample, and cerebrospinal fluid sample, respectively, to obtain solutions of glycosylated exosomes separated from 4 different samples for later use.

### Example 5

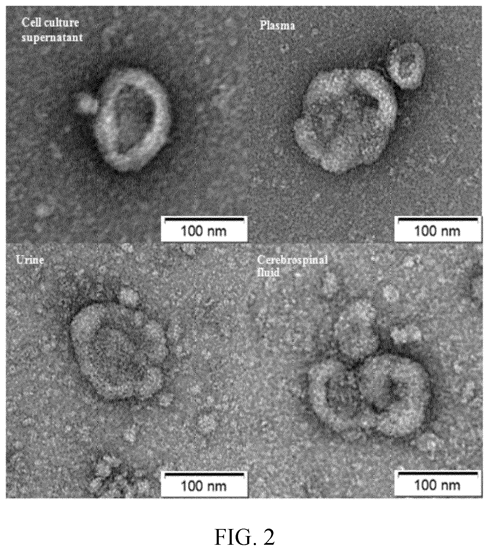

In this example, the glycosylated exosomes separated in Example 4 were subjected to detection by a transmission electron microscope for the morphology and the particle sizes thereof, mainly comprising the steps of:
about 5 uL of the sample solution of glycosylated exosomes separated in Example 4 was dripped onto a copper net for still standing for 4-5 min; excess liquid was sucked off by filter paper from the edge of the copper net; a negative staining fluid (0.5% aqueous uranium acetate solution, pH 4.5) was dripped onto the copper net for reaction thereon for 1 min; the copper net was sucked to dry by the filter paper, was repeatedly washed for 2 times and dried in the air, and then was subjected to observation through Tecnai Spirit (120kV TEM) transmission electron microscope. The observations showed that the glycosylated exosomes separated from the four different types of samples in Example 4 were intact in morphology without rupturing and had the particle sizes ranging from 30 nm to 150 nm. For more details, please see the set of electron microscopic images showing the separation of glycosylated exosomes from 4 different types of samples in Figure 2.

### Example 6

In this example, the glycosylated exosomes separated in Example 4 were subjected to nanoparticle tracking analysis (NTA) by using a NanoSight NS300 system for the granularity and particle sizes thereof; NTA is a test mainly in a solution state (in-situ test), and with its high-precision granularity number distribution test, particles with a relative particle size difference of about 1:1.5 times may be distinguished, which enables exosome particles to be measured in a state closer to an original state, and thereby ensures the authenticity and validity of the measurement; reliable concentration data of the exosomes is provided; the defect that the obtained particle size distribution data is often not representative as a range which can be observed at one time is limited when the exosomes are directly observed and measured by the electron microscope at present is effectively remedied; and damages, observed by the electron microscope, on the exosomes due to pretreatments such as drying, immobilization, freezing, and the like can be effectively lowered.

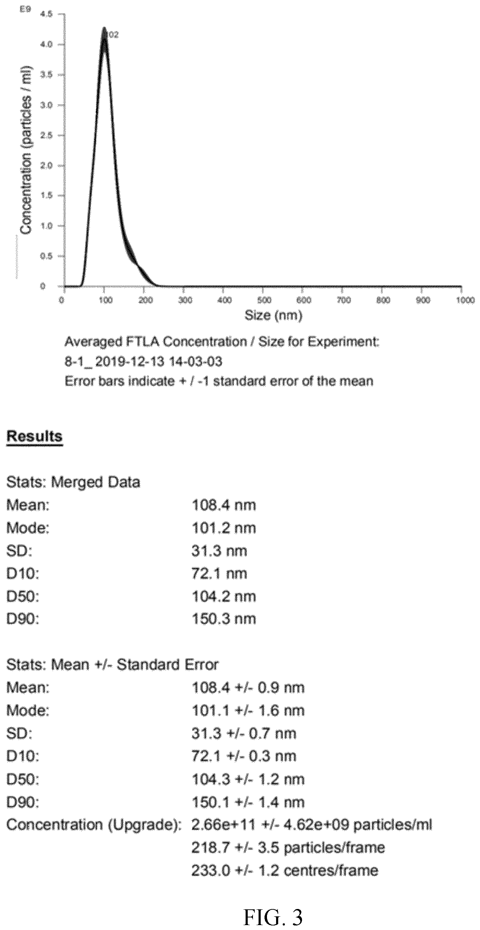

Based on the consistency of results, as observed in Example 5, of 4 different types of samples, in this example, the glycosylated exosomes separated from the plasma sample were selected for NTA detection, comprising the detailed steps of: about 10 uL of sample was diluted to 1 mL; the diluted sample was loaded by a NanoSight injection pump, and was subjected to automatic analysis by the NanoSight NS300 system to obtain the results that the glycosylated exosomes were relatively uniform, had the average particle size ranging from 30 nm to 150 nm, and had the granularity of 10¹¹-10¹⁴/mL. For more details, please see Figure 3, a NTA analysis diagram of glycosylated exosomes separated from a plasma sample.

### Example 7

In this example, the glycosylated exosomes separated in Example 4 were subjected to Western Blot analysis with antibodies against exosome-specific membrane proteins CD9, CD63, and CD81, against exosome-specific non-membrane proteins ALIX and TSG101, and against the protein Calnexin, mainly comprising the following detection steps of:
(1) preparation of a sample: 30 uL of glycosylated exosome eluate was added to an equal volume of 5×SDS-PAGE loading buffer, and the mixture was treated at 100°C for 10 min for later use.
(2) SDS electrophoresis: a gel (15% separation gel, 5% spacer gel) was prepared, 1×electrophoresis buffer was added, 10 µL of sample was added to each hole, and the electrophoresis conditions of 60 V and 120 min were set.
(3) Membrane transfer: a PVDF membrane with a suitable size was placed in a transfer buffer and soaked for 10 min, the gel and the PVDF membrane were closely adhered to a sponge pad to prevent production of bubbles, and proteins were transferred onto the PVDF membrane by using a semi-dry transfer unit (membrane transfer was conducted for 20 min at a constant voltage of 40 V and a constant current of 200 mA).
(4) Blocking: the PVDF membrane was blocked with 5% skim milk powder for 1 h, and then was washed with TBST for 3 times for 5 min each time.
(5) Incubation: the blocked PVDF membrane was incubated for 1 h with primary antibodies (an anti-CD9 monoclonal antibody, an anti-CD81 monoclonal antibody, an anti-CD63 monoclonal antibody, an anti-TSG101 monoclonal antibody, an anti-Alix monoclonal antibody, and an anti-Calnexin monoclonal antibody, with a concentration of 1 mg/mL, and being diluted by 1:400), respectively , followed by a washing step with TBST for 3 times for 5 min each time, and then was incubated with a second antibody (a goat-anti-mouse HRP, 1 mg/mL, diluted by 1:5000) for 1 h, followed by a washing step with TBST for 3 times for 5 min each time.
(6) Development: BeyoECL Moon (very sensitive ECL chemiluminescence kit) was used for color development, and then detection was conducted.

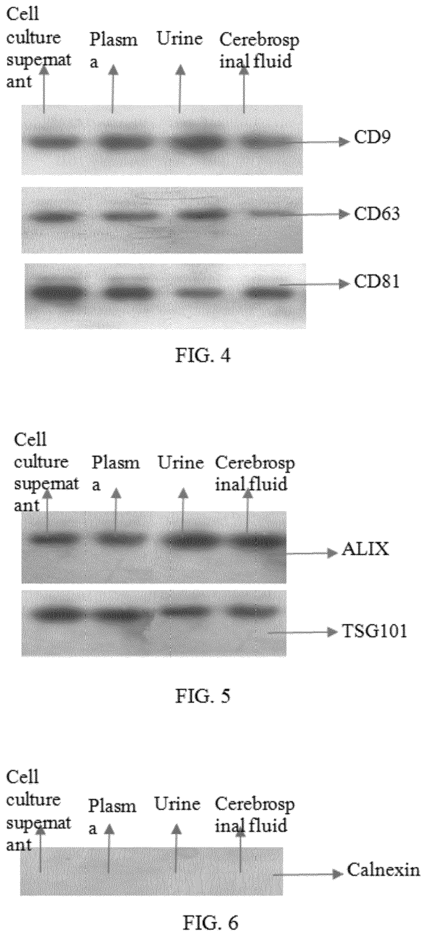

Detection results of the 4 types of glycosylated exosomes separated in Example 4 by Western Blot were as follows:
(1) The Western Blot detections by using antibodies against exosome-specific membrane proteins CD9, CD63, and CD81 were positive, indicating the detection of exosome-specific membrane proteins CD9, CD63, and CD81, and the presence of exosomes in the eluates. For details, please see FIG. 4: a diagram showing results of Western Blot analysis by using the antibodies against the exosome-specific membrane proteins CD9, CD63, and CD81.
(2) The Western Blot detections by using antibodies against exosome-specific non-membrane proteins ALIX and TSG101 were positive, indicating the detection of the exosome-specific non-membrane proteins ALIX and TSG10, and the presence of exosomes in the eluates. For details, please see FIG. 5: a diagram showing results of Western Blot analysis by using the antibodies against the exosome-specific non-membrane proteins ALIX and TSG101.
(3) The Western Blot detection of exosome samples by using an anti-Calnexin antibody was negative, suggesting that the method of the present invention could separate exosomes in a higher purity. The protein Calnexin exists in an endoplasmic reticulum, is a marker protein for the endoplasmic reticulum, but does not exist in exosomes. For details, please see FIG. 6: a diagram showing result of Western Blot analysis of exosomes by using an anti-Calnexin antibody. The glycosylated exosomes separated from the samples in this example were detected to be negative by Western Blot analysis using an anti-Calnexin antibody, indicating that no cell debris existed in the separated glycosylated exosomes.

### Example 8

In this example, the washing and separation effects of different washing buffers, particularly, common washing buffers containing metal salt ions, common washing buffers without metal salt ions, and purified water, in the glycosylated exosome separation process were further compared and verified, so as to determine the suitability of the selected washing buffer in the present invention.

The metal salt ion-free washing buffer (Tris-HCL) in the composition for separating glycosylated exosomes in Examples 2 and 3 was replaced by the purified water and by common washing buffers containing metal salt ions, respectively. The common washing buffers containing metal salt ions comprised: a phosphate buffer (PBS buffer), a Tris-Triton-NaCl buffer, and a TBST buffer. As most of the common washing buffers containing metal salt ions contain surfactants such as Tween-20 and Triton X-100, which can damage outer membranes of the exosome vesicles, when preparing the common washing buffers containing metal salt ions in this example, the component surfactants was excluded therefrom.

In this example, the phosphate buffer (PBS buffer), the Tris-Triton-NaCl buffer without Triton X-100, and the TBST buffer without Tween-20 were respectively used as the common washing buffers containing metal salt ions.

The above PBS buffer comprises the following components: 0.24 g/L of potassium dihydrogen phosphate (KH₂PO4), 1.44 g/L of disodium hydrogen phosphate (Na₂HPO4), 8 g/L of sodium chloride (NaCl), and 0.2 g/L of potassium chloride (KCl), and has a pH value of 7.4±0.2.

The above Tris-Triton-NaCl buffer without Triton X-100 comprises the following components: 50 mM Tris-HCL and 0.6 M NaCL, and has a pH value of 7.6±0.2.

The above TBST buffer without Tween-20 comprises the following components: 10 mM Tris-HCL and 0.15 M NaCL, and has a pH value of 7.6±0.2.

In this example, 5 types of washing buffers including the Tris-Triton-NaCl buffer, the purified water, the PBS buffer, the Tris-Triton-NaCl buffer without Triton X-100, and the TBST buffer without Tween-20 were used, and were marked as Washing Buffer No. 1, Washing Buffer No. 2, Washing Buffer No. 3, Washing Buffer No. 4, and Washing Buffer No. 5, respectively. The 5 types of washing buffers were applied in this example to separate the glycosylated exosomes in a plasma sample, with comparison and verification. In this example, except for the washing buffers, other components and steps are the same. Detailed processes may refer to Examples 1-4, and the detection and identification steps for the exosomes by using antibodies against the exosome-specific membrane proteins CD9, CD63, and CD81 through Western Blot analysis in Example 7.

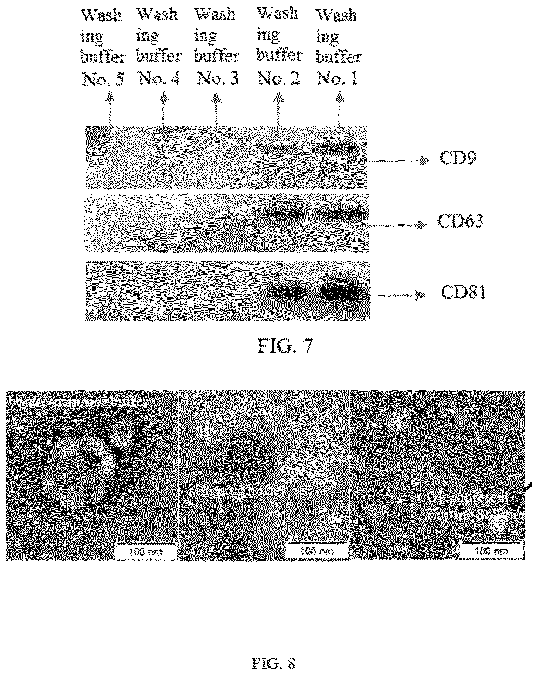

In this example, Western Blot detection results for the glycosylated exosome solutions by using the antibodies against the exosome-specific membrane proteins CD9, CD63, and CD81 were shown in FIG. 7 in detail, wherein the glycosylated exosome solutions were obtained by washing the lectin-magnetic carrier coupling complexes bounded to glycosylated exosomes with the 5 different types of washing buffers and then eluting the same for separation.

Western Blot detection results for eluates, obtained by washing the lectin- magnetic carrier coupling complexes bounded to the glycosylated exosomes with the metal salt ion-free washing buffers (the Washing Buffer No. 1 and the Washing Buffer No. 2) and then eluting the same, show that the exosome-specific membrane proteins CD9, CD63, and CD81 are all positive, indicating that the glycosylated exosomes can be effectively separated with the both metal salt ion-free washing buffers; whereas Western Blot results for eluates, obtained by washing the lectin-magnetic carrier coupling complexes bounded to the glycosylated exosomes with the common washing buffers containing metal salt ions but without the surfactants (the Washing Buffer No. 3, the Washing Buffer No. 4, and the Washing Buffer No. 5) and then eluting the same, showed that the exosome-specific membrane proteins CD9, CD63, and CD81 were all negative, indicating that the metal salt ions in the buffers may affect the separation effect of the glycosylated exosomes and have obvious dissociation effect on the glycosylated exosomes in the present invention.

### Example 9

In this example, the effects of separating glycosylated exosomes by different types of common protein elution buffers and a borate-mannose buffer in the present invention were compared and verified, so as to determine the suitability of the selected elution buffers in the present invention.

The borate-mannose buffer in the composition for separating glycosylated exosomes in Examples 2 and 3 was replaced by a common protein and glycoprotein elution buffer, which included specifically, protein antibody elution buffer (stripping buffer) and glycoprotein elution buffer (Glycoprotein Eluting Solution) (Vectorlabs Company, America).

The above stripping buffer comprises the following components: 62.5 mmol/L Tris·HCl, 2% SDS, and 100 mmol/L β-2-mercaptoethanol, and has a pH value of 6.8±0.2.

The 3 types of elution buffers were applied in this example to separate the glycosylated exosomes in a plasma sample, with comparison and verification. In this example, except for the elution buffer, other components and steps are the same. The detailed processes may refer to Examples 1-5; and the morphology and the integrity of the exosomes of the glycosylated exosomes separated from the plasma sample by the 3 types of elution buffer s were observed through an electron microscope.

In this example, electron microscopic detection results of the glycosylated exosomes separated by using 3 different types of elution buffers were shown in FIG. 8 in detail.

Results showed that the elution buffers in the present invention were able to effectively separate the glycosylated exosomes in a sample, and the separated exosomes were intact in morphology without rupturing and had particle sizes of 30-150 nm; with the stripping buffer as the elution buffer, no exosome was observed, the reason for which may be that the stripping buffer, serving as the elution buffer, may damage outer membranes of the exosomes in the elution process, and thereby no intact exosomes can be obtained; whereas by using the glycoprotein elution buffer (Glycoprotein Eluting Solution), no typcial exosome vesicle was observed, only exosome-like vesicles can be observed, but the morphology thereofwas not intact, indicating that the glycoprotein elution buffer (Glycoprotein Eluting Solution) causes certain damages on the exosomes in the separation process, which goes against subsequent detection or analysis.

To sum up, by implementing the technical solutions of the present invention to separate glycosylated exosomes from different samples, the same effect can be achieved; in addition, the exosomes obtained by the separation are all glycosylated exosomes, and the exosomes are intact in morphology without rupturing after being separated and purified, and may be directly used for liquid detection of glycosylated exosomes, immunological detection of exosomes, or gene detection or analysis after extracting nucleic acids from the exosomes, and may also be subjected to a further purification so as to obtain exosomes with higher purity.

The above description shows and describes the preferred embodiments of the present invention. As previously mentioned, it should be understood that the present invention is not limited to the forms disclosed herein, should not be considered as excluding other embodiments and may be used for various other combinations, modifications and environments; and variations can be made through the above teachings or technologies or knowledges in the related art in the scope of the inventive concept herein.

### Industrial practicability

The present invention relates to a lectin-magnetic carrier coupling complex for separating glycosylated exosomes from a clinical sample. The lectin-magnetic carrier coupling complex comprises a magnetic carrier and lectins coupled to the outer side of the magnetic carrier. The composition provided by the present invention may rapidly, accurately, and automatically separate the glycosylated exosomes from a clinical sample with a high separation efficiency; and the separated exosomes are intact in morphology without rupturing or cracking, and may be directly used for detection of glycosylated exosome liquid, or directly used for immunology-related detection, or directly used for nucleotide sequence detection or analysis after extracting nucleic acids from exosomes.

## Claims

1. A composition for separating glycosylated exosomes, comprising a lectin-magnetic carrier coupling complex, a washing buffer for washing and for removing the exosomes nonspecifically bound to the coupling complex or not glycosylated and other impurities in the separation process, and an elution buffer for eluting the glycosylated exosomes specifically bound to the lectin-magnetic carrier coupling complex;
wherein,
the lectin-magnetic carrier coupling complex comprises a magnetic carrier and lectins coupled to the outer side of the magnetic carrier, wherein
the lectins are any one type of Artocarpus integrifolia lectin, peanut lectin, Pisum sativum lectin, Concanavalin lectin, Lens culinaris lectin, wheat germ lectin, soybean lectin, kidney bean lectin, and snail lectin, or a combination of two or more of the above; and
the magnetic carrier is any one type of a dextran magnetic bead, an agarose magnetic bead, a resin or epoxy resin magnetic bead, and a polystyrene magnetic bead, or a combination of two or more of the above; and
the magnetic carrier has a particle size distribution range from 1 µm to 200 µm;
the washing buffer is a metal salt ion-free washing buffer or purified water, and optionally, is a metal salt ion-free washing buffer; and
the elution buffer is a borate buffer with saccharides dissolved therein, and optionally, is a borate buffer with mannose dissolved therein.

2. The composition for separating glycosylated exosomes according to claim 1, wherein in the lectin-magnetic carrier coupling complex, the magnetic carrier has a particle size distribution range from 10 µm to 200 µm.

3. The composition for separating glycosylated exosomes according to claim 2, wherein in the lectin-magnetic carrier coupling complex, the magnetic carrier has a particle size distribution range from 30 µm to 150 µm.

4. The composition for separating glycosylated exosomes according to claim 1, wherein 1-20 mg of the lectins are coupled to each l mL of the magnetic carrier.

5. The composition for separating glycosylated exosomes according to claim 4, wherein 5-15 mg of the lectins are coupled to each 1 mL of the magnetic carrier.

6. The composition for separating glycosylated exosomes according to claim 5, wherein 10-15 mg of the lectins are coupled to each l mL of the magnetic carrier.

7. The composition for separating glycosylated exosomes according to claim 1, wherein in the lectin-magnetic carrier coupling complex, the lectins are any one type of Artocarpus integrifolia lectin, peanut lectin, Pisum sativum lectin (VVA and/or VVL), Concanavalin lectin, Lens culinaris lectin, wheat germ lectin, soybean lectin, and kidney bean lectin, or a combination of two or more of the above.

8. The composition for separating glycosylated exosomes according to claim 1, wherein the lectin-magnetic carrier coupling complex is used for separating glycosylated exosomes from a clinical sample, wherein the clinical sample is any one of serum, plasma, saliva, a tissue or cell culture supernatant, urine, a cerebrospinal fluid, and a lymph fluid, and/or the glycosylated exosomes are any one type of N-glycosylated exosomes, O-glycosylated exosomes, and fucosylated exosomes, or a combination of two or more of the above.

9. A method of separating glycosylated exosomes, comprising a separation step of using the composition for separating glycosylated exosomes according to any one of claims 1-8 to separate glycosylated exosomes, wherein the separation step comprises:
pretreatment of a clinical sample:
fully and uniformly mixing the pretreated sample with the lectin-magnetic carrier coupling complex for incubation;
conducting separation on the lectin-magnetic carrier coupling complex bound to the glycosylated exosomes by means of magnetic separation, and washing the lectin-magnetic carrier coupling complex bound to the glycosylated exosomes with the washing buffer; and eluting the washed lectin-magnetic carrier coupling complex with the elution buffer.

10. The method of separating glycosylated exosomes according to claim 9, wherein a mixing ratio by volume of the pretreated sample to the lectin-magnetic carrier coupling complex is (0.5-5): 1; and/or the washing step is performed with the washing buffer 1-3 times the volume of the lectin-magnetic carrier coupling complex for 1-3 times;
and/or a ratio by volume of the elution buffer to the lectin-magnetic carrier coupling complex is (0.5-2): 1.

11. The method of separating glycosylated exosomes according to claim 10, wherein a mixing ratio by volume of the pretreated sample to the lectin-magnetic carrier coupling complex is (0.5-3): 1;
and/or a ratio by volume of the elution buffer to the lectin-magnetic carrier coupling complex is (0.5-1): 1.

12. The method of separating glycosylated exosomes according to claim 11, wherein a mixing ratio by volume of the pretreated sample to the lectin-magnetic carrier coupling complex is (1-2): 1.

13. The method of separating glycosylated exosomes according to claim 9, wherein the pretreatment of the sample comprises the following steps:
for a serum sample, a plasma sample, a saliva sample, a cerebrospinal fluid sample, or a lymph fluid sample, subjecting the sample to centrifugation at a speed of 3000 g or below for 10-15 min, to remove cell debris, precipitates, and other impurities in the sample, and taking the supernatant after centrifugation for later use; and
for a tissue or cell culture supernatant sample or a urine sample, subjecting the sample to centrifugation at a speed of 3000 g or below for 10-15 min, to remove cell debris, precipitates, and other impurities in the sample, and then concentrating the supernatant by 10-1000 times through an ultrafiltration tube for later use.

14. The method of separating glycosylated exosomes according to claim 9, wherein
the incubation is conducted under the conditions of: at room temperature for 1-30 min.

15. The method of separating glycosylated exosomes according to claim 14, wherein the incubation is conducted under the conditions of: at room temperature for 5-20 min.

16. The method of separating glycosylated exosomes according to claim 15, wherein the incubation is conducted under the conditions of: at room temperature for 10-15 min.

## Patentansprüche

1. Zusammensetzung zur Isolierung von glykosylierten Exosomen, aufweisend einen Lektin-Magnetträger-Konjugatkomplex, einen Waschpuffer zum Waschen und zum Entfernen der nicht spezifisch an den Konjugatkomplex gebundenen oder nicht glykosylierten Exosomen und anderer Verunreinigungen im Isolierprozess, und einen Elutionspuffer zum Eluieren der spezifisch an den Lektin-Magnetträger-Konjugatkomplex gebundenen glykosylierten Exosomen;
wobei,
der Lektin-Magnetträger-Konjugatkomplex einen magnetischen Träger und an die Außenseite des magnetischen Trägers konjugierte Lektine aufweist, wobei die Lektine ein beliebiger Typ von Artocarpus-integrifolia-Lektin, Erdnuss-Lektin, Pisum-sativum-Lektin, Concanavalin-Lektin, Lens-culinaris-Lektin, Weizenkeim-Lektin, Sojabohnen-Lektin, Kidneybohnen-Lektin und Schnecken-Lektin oder eine Kombination von zwei oder mehreren der oben genannten sind; und
der magnetische Träger ist ein beliebiger Typ eines Dextran-Magnetkügelchens, eines Agarose-Magnetkügelchens, eines Harz- oder Epoxidharz-Magnetkügelchens und eines Polystyrol-Magnetkügelchens oder eine Kombination von zwei oder mehreren der oben genannten; und
der magnetische Träger eine Partikelgrößenverteilung im Bereich von 1 µm bis 200 µm hat;
der Waschpuffer ein Metallsalzionen-freier Waschpuffer oder gereinigtes Wasser ist und gegebenenfalls ein Metallsalzionen-freier Waschpuffer ist; und
der Elutionspuffer ein Boratpuffer mit darin gelösten Sacchariden ist, und gegebenenfalls ein Boratpuffer mit darin gelöster Mannose ist.

2. Zusammensetzung zur Isolierung von glykosylierten Exosomen nach Anspruch 1, wobei der magnetische Träger in dem Lektin-Magnetträger-Konjugatkomplex eine Teilchengrößenverteilung im Bereich von 10 µm bis 200 µm aufweist.

3. Zusammensetzung zur Isolierung von glykosylierten Exosomen nach Anspruch 2, wobei der magnetische Träger in dem Lektin-Magnetträger-Konjugatkomplex eine Partikelgrößenverteilung im Bereich von 30 µm bis 150 µm aufweist.

4. Zusammensetzung zur Isolierung von glykosylierten Exosomen nach Anspruch 1, wobei 1-20 mg der Lektine an je 1 ml des magnetischen Trägers konjugiert sind.

5. Zusammensetzung zur Isolierung von glykosylierten Exosomen nach Anspruch 4, wobei 5-15 mg der Lektine an jeweils 1 ml des magnetischen Trägers konjugiert sind.

6. Zusammensetzung zur Isolierung von glykosylierten Exosomen nach Anspruch 5, wobei 10-15 mg der Lektine an jeweils 1 ml des magnetischen Trägers konjugiert sind.

7. Zusammensetzung zur Isolierung von glykosylierten Exosomen nach Anspruch 1, wobei in dem Lektin-Magnetträger-Konjugatkomplex die Lektine ein beliebiger Typ von Artocarpus integrifolia-Lektin, Erdnuss-Lektin, Pisum sativum-Lektin (WA und/oder WL), Concanavalin-Lektin, Lens culinaris-Lektin, Weizenkeim-Lektin, Sojabohnen-Lektin und Kidneybohnen-Lektin oder eine Kombination von zwei oder mehreren der oben genannten sind.

8. Zusammensetzung zur Isolierung von glykosylierten Exosomen nach Anspruch 1, wobei der Lektin-Magnetträger-Konjugatkomplex zur Isolierung von glykosylierten Exosomen aus einer klinischen Probe verwendet wird, wobei die klinische Probe eines der folgenden ist: Serum, Plasma, Speichel, ein Gewebe- oder Zellkulturüberstand, Urin, eine zerebrospinale Flüssigkeit und eine Lymphflüssigkeit ist, und/oder die glykosylierten Exosomen ein beliebiger Typ von N-glykosylierten Exosomen, O-fucosylierten Exosomen und glykosylierten Exosomen oder eine Kombination von zwei oder mehreren der oben genannten sind.

9. Verfahren zur Isolierung von glykosylierten Exosomen, umfassend einen Isolierungsschritt der Verwendung der Zusammensetzung zur Isolierung von glykosylierten Exosomen gemäß einem der Ansprüche 1-8, um glykosylierte Exosomen zu isolieren, wobei der Isolierungsschritt umfasst:
Vorbehandeln einer klinischen Probe:
vollständiges und gleichmäßiges Mischen der vorbehandelten Probe mit dem Lektin-magnetischen Träger-Konjugatkomplex zur Inkubation;
Durchführen einer Isolierung des an die glykosylierten Exosomen gebundenen Lektin-Magnet-Träger-Konjugatkomplexes mittels magnetischer Isolierung und Waschen des an die glykosylierten Exosomen gebundenen Lektin-Magnet-Träger-Konjugatkomplexes mit dem Waschpuffer; und
Eluieren des gewaschenen Lektin-Magnetträger-Konjugatkomplex mit dem Elutionspuffer.

10. Verfahren zur Isolierung von glykosylierten Exosomen nach Anspruch 9, wobei das volumenbezogene Mischungsverhältnis der vorbehandelten Probe zu dem Lektin-Magnetträger-Konjugatkomplex (0,5-5): 1 ist, und/oder der Waschschritt mit dem 1-3-fachen Volumen des Lektin-Magnet-Träger-Konjugatkomplexes 1-3-mal mit dem Waschpuffer durchgeführt wird;
und/oder ein Volumenverhältnis des Elutionspuffers zu dem Lektin-magnetischen Trägerkupplungskomplex (0,5-2): 1 ist.

11. Verfahren zur Isolierung von glykosylierten Exosomen nach Anspruch 10, wobei das volumenbezogene Mischungsverhältnis der vorbehandelten Probe zu dem Lektin-Magnetträger-Konjugatkomplex (0,5-3): 1 ist;
und/oder ein Volumenverhältnis des Elutionspuffers zu dem Lektin-Magnetträger-Konjugatkomplex (0,5-1): 1 ist.

12. Verfahren zur Isolierung von glykosylierten Exosomen nach Anspruch 11, wobei das volumenbezogene Mischungsverhältnis der vorbehandelten Probe zu dem Lektin-Magnetträger-Konjugatkomplex (1-2): 1 ist.

13. Verfahren zur Isolierung von glykosylierten Exosomen nach Anspruch 9, wobei die Vorbehandlung der Probe die folgenden Schritte umfasst:
bei einer Serum-, Plasma-, Speichel-, Liquor- oder Lymphflüssigkeitsprobe:
Unterziehen der Probe einer Zentrifugation bei einer Geschwindigkeit von 3000 g oder weniger für 10-15 min, um Zelltrümmer, Präzipitate und andere Verunreinigungen in der Probe zu entfernen, und Entnahme des Überstandes nach der Zentrifugation zur späteren Verwendung; und
bei einer Gewebe- oder Zellkulturüberstandsprobe oder einer Urinprobe, Unterziehen der Probe einer Zentrifugation bei einer Geschwindigkeit von 3000 g oder weniger für 10-15 min, um Zelltrümmer, Präzipitate und andere Verunreinigungen in der Probe zu entfernen, und anschließendes Konzentrieren des Überstands um das 10-1000-fache durch ein Ultrafiltrationsrohr zur späteren Verwendung.

14. Verfahren zur Isolierung von glykosylierten Exosomen nach Anspruch 9, wobei die Inkubation unter den folgenden Bedingungen durchgeführt wird: bei Raumtemperatur für 1-30 min.

15. Verfahren zur Isolierung von glykosylierten Exosomen nach Anspruch 14, wobei die Inkubation unter den folgenden Bedingungen durchgeführt wird: bei Raumtemperatur für 5-20 Minuten.

16. Verfahren zur Isolierung von glykosylierten Exosomen nach Anspruch 15, wobei die Inkubation unter den folgenden Bedingungen durchgeführt wird: bei Raumtemperatur für 10-15 Minuten.

## Revendications

1. Une composition pour séparer des exosomes glycosylés, comprenant un complexe de liaison lectine-support magnétique, un tampon de lavage pour laver et pour éliminer les exosomes non-spécifiquement liés au complexe de liaison ou non-glycosylés et d'autres impuretés dans le processus de séparation, et un tampon d'élution pour éluer les exosomes glycosylés spécifiquement liés au complexe de liaison lectine-support magnétique ;
dans laquelle
le complexe de liaison lectine-support magnétique comprend un support magnétique et des lectines reliées au côté extérieur du support magnétique,
les lectines sont un type quelconque de lectine d'Artocarpus integrifolia, de lectine d'arachide, de lectine de Pisum sativum, de lectine de Concanavaline, de lectine de Lens culinaris, de lectine de germe de blé, de lectine de soja, de lectine de haricot rouge et de lectine d'escargot, ou une combinaison de deux ou plusieurs des éléments ci-dessus ; et le support magnétique est un type quelconque de bille magnétique de dextrane, de bille magnétique d'agarose, de bille magnétique de résine ou de résine époxy et de bille magnétique de polystyrène, ou une combinaison de deux ou plusieurs des éléments ci-dessus ; et
le support magnétique a une gamme de distribution granulométrique comprise entre 1µm et 200µm ;
le tampon de lavage est un tampon de lavage dépourvu d'ions de sel métallique ou de l'eau purifiée, et optionnellement, est un tampon de lavage dépourvu d'ions de sel métallique ; et
le tampon d'élution est un tampon de borate contenant des saccharides dissous en lui, et optionnellement, est un tampon de borate contenant du mannose dissous en lui.

2. La composition pour séparer des exosomes glycosylés selon la revendication 1, dans laquelle dans le complexe de liaison lectine-support magnétique, le support magnétique a une gamme de distribution granulométrique comprise entre 10µm et 200µm.

3. La composition pour la séparation d'exosomes glycosylés selon la revendication 2, dans laquelle dans le complexe de liaison lectine-support magnétique, le support magnétique a une distribution granulométrique comprise entre 30µm et 150µm.

4. La composition pour la séparation d'exosomes glycosylés selon la revendication 1, dans laquelle 1 à 20mg de lectines sont reliées à chaque 1mL du support magnétique.

5. La composition pour la séparation d'exosomes glycosylés selon la revendication 4, dans laquelle 5 à 15mg de lectines sont reliées à chaque 1mL du support magnétique.

6. La composition pour la séparation d'exosomes glycosylés selon la revendication 5, dans laquelle 10 à 15mg de lectines sont reliées à chaque 1mL du support magnétique.

7. La composition pour la séparation d'exosomes glycosylés selon la revendication 1, dans laquelle dans le complexe de liaison lectine-support magnétique, les lectines sont un type quelconque de lectine d'Artocarpus integrifolia, de lectine d'arachide, de lectine de Pisum sativum (VVA et/ou VVL), de lectine de concanavaline, de lectine de Lens culinaris, de lectine de germe de blé, de lectine de soja et de lectine de haricot rouge, ou une combinaison de deux ou plusieurs des lectines ci-dessus.

8. La composition pour la séparation d'exosomes glycosylés selon la revendication 1, dans laquelle le complexe de liaison lectine-support magnétique est utilisé pour séparer des exosomes glycosylés d'un échantillon clinique, l'échantillon clinique étant l'un quelconque parmi le sérum, le plasma, la salive, un surnageant de culture tissulaire ou cellulaire, l'urine, un liquide céphalo-rachidien et un liquide lymphatique, et/ou les exosomes glycosylés sont l'un quelconque des types d'exosomes N-glycosylés, d'exosomes O-glycosylés et d'exosomes fucosylés, ou une combinaison de deux ou plusieurs des éléments ci-dessus.

9. Un procédé de séparation d'exosomes glycosylés, comprenant une étape de séparation consistant à utiliser la composition de séparation d'exosomes glycosylés selon l'une quelconque des revendications 1 à 8 pour séparer des exosomes glycosylés, l'étape de séparation comprenant :
le fait de prétraiter un échantillon clinique ;
le fait de mélanger complètement et uniformément l'échantillon prétraité avec le complexe de liaison lectine-support magnétique pour incubation ;
le fait de réaliser une séparation sur le complexe de liaison lectine-support magnétique lié aux exosomes glycosylés au moyen d'une séparation magnétique, et le fait de laver le complexe de liaison lectine-support magnétique lié aux exosomes glycosylés avec le tampon de lavage ; et
le fait d'éluer le complexe de liaison lectine-support magnétique lavé avec le tampon d'élution.

10. Le procédé de séparation d'exosomes glycosylés selon la revendication 9, dans lequel un rapport de mélange en volume de l'échantillon prétraité au complexe de liaison lectine-support magnétique est (0,5-5) : 1 ;
et/ou l'étape de lavage est réalisée 1 à 3 fois avec le tampon de lavage 1 à 3 fois le volume du complexe de liaison lectine-support magnétique ;
et/ou un rapport en volume du tampon d'élution au complexe de liaison lectine-support magnétique est (0,5-2) : 1.

11. Le procédé de séparation d'exosomes glycosylés selon la revendication 10, dans lequel un rapport de mélange en volume de l'échantillon prétraité au complexe de liaison lectine-support magnétique est (0,5-3) : 1 ;
et/ou un rapport en volume du tampon d'élution au complexe de liaison lectine-support magnétique est (0,5-1) : 1.

12. Le procédé de séparation d'exosomes glycosylés selon la revendication 11, dans lequel un rapport de mélange en volume de l'échantillon prétraité au complexe de liaison lectine-support magnétique est (1-2) : 1.

13. Le procédé de séparation d'exosomes glycosylés selon la revendication 9, dans lesquels le prétraitement de l'échantillon comprend les étapes suivantes :
pour un échantillon de sérum, un échantillon de plasma, un échantillon de salive, un échantillon de liquide céphalorachidien ou un échantillon de liquide lymphatique, le fait de soumettre l'échantillon à une centrifugation à une vitesse de 3000 g ou moins pendant 10 à 15 min, afin d'éliminer les débris cellulaires, les précipités et autres impuretés de l'échantillon, et le fait de prélever le surnageant après centrifugation pour une utilisation ultérieure ; et
pour un échantillon de surnageant de culture tissulaire ou cellulaire ou un échantillon d'urine, le fait de soumettre l'échantillon à une centrifugation à une vitesse de 3000 g ou moins pendant 10 à 15 min, afin d'éliminer les débris cellulaires, les précipités et
autres impuretés de l'échantillon, puis le fait de concentrer le surnageant de 10 à 1000 fois à travers un tube d'ultrafiltration pour une utilisation ultérieure.

14. Le procédé de séparation d'exosomes glycosylés selon la revendication 9, dans lequel l'incubation est réalisée dans les conditions suivantes : à température ambiante pendant 1 à 30 min.

15. Le procédé de séparation d'exosomes glycosylés selon la revendication 14, dans lequel l'incubation est réalisée dans les conditions suivantes : à température ambiante pendant 5 à 20 min.

16. Le procédé de séparation d'exosomes glycosylés selon la revendication 15, dans lequel l'incubation est réalisée dans les conditions suivantes : à température ambiante pendant 10 à 15 min.
